# EUROPÄISCHE PATENTSCHRIFT

(11) **EP 4 085 963 B1**
(45) Veröffentlichungstag und Bekanntmachung des Hinweises auf die Patenterteilung: **12.03.2025**
(21) Anmeldenummer: 21172237.6
(22) Anmeldetag: 05.05.2021
(51) Int. Cl.: A61M 37/00, A01K 11/00, F16H 23/02, H02K 7/075

(54) **ANTRIEBSEINRICHTUNG FÜR EIN HANDGERÄT ZUM LOKALEN AUFSTECHEN EINER HAUT UND HANDGERÄT**
DRIVING DEVICE FOR A HAND TOOL FOR THE LOCAL PIERCING OF A SKIN AND HAND-HELD DEVICE
DISPOSITIF D'ENTRAÎNEMENT POUR UN APPAREIL PORTATIF DE PERÇAGE LOCAL DE LA PEAU ET APPAREIL PORTATIF

(43) Veröffentlichungstag der Anmeldung: 09.11.2022
(73) Patentinhaber: MT.DERM GmbH, 14167 Berlin (DE)
(72) Erfinder: SCHERKOWSKI, Dirk, 12489 Berlin (DE)
(74) Vertreter: Bittner, Thomas L.

(56) Entgegenhaltungen:
- CN-A- 108 635 665
- US-A1- 2005 010 236
- US-A1- 2020 324 098

## Beschreibung

Die Erfindung betrifft eine Antriebseinrichtung für ein Handgerät zum lokalen Aufstechen einer Haut sowie ein Handgerät zum lokalen Aufstechen einer Haut.

### Hintergrund

Solche Handgeräte dienen dazu, eine menschliche oder eine tierische Haut lokal aufzustechen, insbesondere in Verbindung mit dem Tätowieren oder zum Ausbilden von permanentem Makeup. Hierbei wird beim und / oder nach dem lokalen Aufstechen der Haut ein Farbstoff in die Haut eingebracht. Das lokale Aufstechen der Haut mit einem solchen Handgerät kann aber auch zum Einbringen kosmetischer oder medizinischer Wirkstoffe verwendet werden. Alternativ ist die Verwendung derartiger Handgeräte zum lokalen Aufstechen der Haut ohne Substanz- oder Wirkstoffeintrag bekannt, zum Beispiel zur Hautstimulierung.

Bei Handgeräten zum lokalen Aufstechen der Haut ist üblicherweise eine Antriebseinrichtung mit einem elektrischen Motor vorgesehen, mit dem eine drehende Antriebsbewegung bereitgestellt wird. Die Antriebsbewegung wird mit Hilfe einer Kopplungseinrichtung abgegriffen und in eine Linearbewegung umgewandelt, die auf eine Stecheinrichtung eingekoppelt wird, um eine oder mehrere Stechnadeln im Betrieb mit einer Wiederholfrequenz vor und zurück zu bewegen, um die Haut lokal aufzustechen.

Das Dokument CN 108 635 665 A offenbart ein geräuscharmes Tattoo-Auffrischungsinstrument, das ein Bedienfeld vom Uhrentyp, einen Bedienstift, einen starken magnetischen Verbinder und eine Augenbrauen-Tätowierungsnadel umfasst, wobei das Bedienfeld vom Uhrentyp mit dem Bedienstift verbunden ist, der Bedienstift mit einem Hohlschalenmotor installiert ist, der Hohlschalenmotor mit der Augenbrauen-Tätowierungsnadel an der Vorderseite des Bedienstifts verbunden ist und das Bedienfeld vom Uhrentyp mit einem Einschaltknopf und einem Leistungseinstellknopf installiert ist.

Dokument US 2005 / 0 010 236 A1 betrifft eine Vorrichtung zum Aufbringen einer Tätowierung oder von permanentem Make-up, mit einem Einwegmodul, welches lösbar an einem Handstück angeordnet ist, einer Nadel, die mit einem Ende in einem Nadelschaft gehalten wird, die bewegbar in einer an dem Einwegmodul gebildeten Nadelführung gelagert ist und die mit einem anderen Ende durch eine Nadeldüsenöffnung zum Ausbringen eines Farbstoffs verläuft, und einem an den Nadelschaft zum Vor- und Zurückbewegen der Nadel gekoppelten und von mehreren Bauteilen gebildeten Antriebsmechanismus, wobei die mehreren Bauteile des Antriebsmechanismus einen Antrieb und Kopplungsmittel zum Koppeln des Antriebs an den Nadelschaft umfassen. Zumindest ein Teil der mehreren Bauteile des Antriebsmechanismus ist in dem Einwegmodul angeordnet und zusammen mit dem Einwegmodul von dem Handstück abnehmbar ist.

Das Dokument US 2020 / 0 324 098 A1 betrifft ein Antriebssystem für ein Handgerät zum lokalen Aufstechen einer menschlichen oder einer tierischen Haut, mit einer Antriebseinrichtung, die eingerichtet ist, eine drehende Antriebsbewegung bereitzustellen; und einem Wandlungsmechanismus, weicher zum Abgreifen der drehenden Antriebsbewegung an die Antriebseinrichtung koppelt und eingerichtet ist, ausgehend von der drehenden Antriebsbewegung eine lineare Vorwärts- und Rückwärtsbewegung bereitzustellen, der Wandlungsmechanismus aufweisend: eine Taumelscheibenanordnung, die an einer Lagereinrichtung angeordnet ist und über ein Drehgelenk an ein distales Gelenkbauteil koppelt, welches von der Lagereinrichtung aufgenommen ist; eine an der Taumelscheibenanordnung gebildete Taumelscheibe, welche im Betrieb aufgrund der drehenden Antriebsbewegung eine Taumelbewegung ausführt; einen Abgriff, weicher an die Taumelscheibe koppelt und eingerichtet ist, mittels Zusammenwirken mit der Taumelscheibe die lineare Vorwärts- und Rückwärtsbewegung zum Abgreifen bereitzustellen; und ein Einstellbauteil, welches an die Taumelscheibenanordnung koppelt und verlagerbar angeordnet ist, derart, dass zum Einstellen eines Hubs der an dem Abgriff bereitgestellten linearen Vorwärts- und Rückwärtsbewegung eine Neigung der Taumelscheibe zur Längsrichtung mittels Verlagern des Einstellbauteils veränderbar ist.

### Zusammenfassung

Aufgabe der Erfindung ist es, eine Antriebseinrichtung für ein Handgerät zum lokalen Aufstechen einer Haut sowie ein solches Handgerät zu schaffen, mit denen eine Antriebsbewegung für eine Stecheinrichtung effizient und für unterschiedliche Anwendungen flexibel bereitgestellt werden kann.

Zur Lösung sind eine Antriebseinrichtung für ein Handgerät zum lokalen Aufstechen einer Haut sowie ein Handgerät zum lokalen Aufstechen einer Haut nach den unabhängigen Ansprüchen 1 und 12 geschaffen. Ausgestaltungen sind Gegenstand von abhängigen Unteransprüchen. Nach einem Aspekt ist eine Antriebseinrichtung für ein Handgerät zum lokalen Aufstechen einer Haut geschaffen, die Folgendes aufweist: ein Gehäuse, welches mit einem Modulgehäuse einer Hautstecheinrichtung verbindbar ist; und eine elektrische Antriebsvorrichtung, die in dem Gehäuse angeordnet ist und ein Abgriffbauteil aufweist, welches eingerichtet ist, einen Abgriff zum Abgreifen einer mittels der elektrischen Antriebsvorrichtung erzeugten drehenden Antriebsbewegung für die Hautstecheinrichtung bereitzustellen. Die elektrische Antriebsvorrichtung weist einen als Außenläufer mit einem rotierenden Rotorbauteil ausgebildeten elektrischen Motor auf, bei dem das Abgriffbauteil an dem Rotorbauteil und in Bezug auf eine Rotorachse exzentrisch angeordnet ist.

Nach einem weiteren Aspekt ist ein Handgerät zum lokalen Aufstechen einer Haut mit einer Antriebseinrichtung sowie einer Hautstecheinrichtung mit einem Modulgehäuse geschaffen, welche mit der Antriebseinrichtung lösbar verbunden ist und bei der im Betrieb eine Stecheinrichtung in einer vorderseitigen Modulgehäuseöffnung vor und zurückbewegt werden kann. Das Handgerät umfasst eine Kopplungseinrichtung, die als Teil der Antriebseinrichtung und / oder der Hautstecheinrichtung gebildet und eingerichtet ist, eine in der Antriebseinrichtung bereitgestellte drehende Antriebsbewegung am Abgriff eines als Außenläufer ausgebildeten elektrischen Motors abzugreifen und in eine Linearbewegung für die Stecheinrichtung auf diese zu koppeln.

Es ist vorgesehen, den Abgriff am elektrischen Motor getrennt von der Motorwelle an dem Rotorbauteil des elektrischen Motors auszubilden. Es ist so vermieden, zum Ausbilden einer Exzentrizität des Abgriffs in Bezug auf die Rotor- oder Motorachse des elektrischen Motors zusätzliche Bauteile auf der Motorwelle zu montieren, an denen dann der Abgriff gebildet ist. Auf diese Weise kann die Antriebseinrichtung mit verbesserter mechanischer Stabilität ausgebildet werden.

Mittels Anordnen des Abgriffs auf dem Rotorbauteil des Außenläufers entfällt die sonst beim Stand der Technik notwendige Umhüllung einer Motorwelle durch ein üblicherweise zylindrisches, exzentrisch angeordnetes Abgriffbauteil auf der Motorwelle. Das am Rotorbauteil (nicht auf der Motorwelle) angeordnete Abgriffbauteil kann so hinsichtlich seiner Abmessungen kleiner gewählt werden, bis wahlweise hin zu einer Größe kleiner als die Motorwelle, zum Beispiel betreffend einen Durchmesser. Der am Rotorbauteil selbst vorgesehene Abgriff wird allein durch ein Biegemoment beansprucht und kann daher kleiner gehalten werden.

Mit der Kopplungseinrichtung ist ein Wandlungsmechanismus bereitgestellt oder implementiert, mit dem die drehende Antriebsbewegung in die Linearbewegung gewandelt wird, wie dies als solches für Handgeräte zum lokalen Aufstechen einer Haut in verschiedenen Ausgestaltungen bekannt ist. Die Einkopplung der Antriebsbewegung auf die Stecheinrichtung kann derart ausgeführt sein, dass die Stecheinrichtung hierdurch zwangsweise vor- und / oder zurückbewegt wird. Alternativ oder ergänzend können andere oder weitere Kräfte auf die Stecheinrichtung eingekoppelt werden, die deren Bewegung beim Vor- und / oder Zurückbewegen bewirken oder unterstützen, beispielsweise eine Federkraft uns / oder eine von einer gestreckten Membran aufgebrachte Kraft, wie dies als solches ebenso in verschiedenen Ausführungen bekannt ist.

Mit dem Ausbilden des Abgriffbauteils an dem Rotorbauteil selbst kann ein exzentrischer Kurbelzapfen bereitgestellt sein, an dem die drehende Antriebsbewegung abgegriffen werden kann. In einer Ausführungsform kann das Rotorbauteil, an dem das Abgriffbauteil angeordnet ist, eine rotierende Außenglocke oder eine rotierendes Gehäusebauteil des elektrischen Motors sein.

Es kann vorgesehen sein, den elektrischen Motor frei von einem Motorwellenkoppelzapfen auszubilden, da der Abgriff der drehenden Antriebsbewegung über das Abgriffbauteil an dem Rotorbauteil erfolgt. Der elektrische Motor kann so frei von einer äußeren Motorwelle sein.

Das Abgriffbauteil kann auf einer Stirnfläche des Rotorbauteils angeordnet sein. Alternativ kann das Abgriffbauteil im Bereich einer Umfangsfläche des Rotorbauteils oder im Bereich eines Übergangs zwischen der Stirnfläche und der Umfangsfläche angeordnet sein.

Das Abgriffbauteil kann an dem Rotorbauteil verlagerbar angeordnet sein, derart, dass eine Exzentrizität des Abgriffs in Bezug auf die Rotorachse einstellbar ist. Die Verlagerbarkeit des Abgriffbauteils an dem Rotorbauteil ist insbesondere in radialer Richtung gegeben, so dass ein Abstand zwischen dem den Abgriff bereitstellenden Abgriffbauteil und der Rotor- oder Motorachse vergrößert und verkleinert werden kann. Hierzu kann das Abgriffbauteil beispielsweise mittels einer lösbaren Klemm- oder Schraubverbindung an dem Rotorbauteil montiert sein, die gelöst werden kann, um das Abgriffbauteil an dem Rotorbauteil zu verlagern. Nach der Verlagerung kann die Verbindung wieder festgestellt werden, um das Abgriffbauteil an dem Rotorbauteil in der neuen Stellung zu fixieren. Mit Hilfe einer Änderung der Exzentrizität kann beim Koppeln der Antriebseinrichtung an die Hautstecheinrichtung ein Hub für die Stecheinrichtung (Länge der Vor- und Zurückbewegung; maximaler Abstand zwischen Umkehrpunkten einer linearen Stechbewegung) eingestellt werden, insbesondere eines Nadelhubs einer Nadeleinrichtung der Hautstecheinrichtung, die eine oder mehrere Stechnadeln aufweist, welche zum lokalen Aufstechen der Haut vor und zurück bewegt werden.

Das Abgriffbauteil kann an dem Rotorbauteil einstückig angeformt sein. Zum einstückigen Anformen des Abgriffbauteils an dem Rotorbauteil kann vorgesehen sein, zwischen den Bauteilen zum Beispiel eine Kleb- oder eine Schweißverbindung auszubilden.

Das Abgriffbauteil kann an dem Rotorbauteil lösbar angeordnet sein. Eine lösbare Verbindung zwischen Abgriffbauteil und Rotorbauteil kann beispielsweise mittels einer Schraub- oder eine Steckverbindung ausgebildet sein.

Dem Abgriffbauteil kann eine Ausgleichsmasse zugeordnet sein. Mit Hilfe der dem Abgriffbauteil zugeordneten Ausgleichsmasse werden Vibrationen im Betrieb der Antriebseinrichtung vermindert oder ganz beseitigt.

Die Ausgleichsmasse kann mit einem Ausgleichsbauteil gebildet sein, welches an dem Rotorbauteil montiert oder angeordnet ist. Das Ausgleichsbauteil kann verlagerbar an dem Rotorbauteil angeordnet sein. Auf diese Weise ist eine Einstellbarkeit der Ausgleich- oder Auswuchtwirkung ermöglicht, beispielsweise dann, wenn die Exzentrizität für den Abgriff an dem Rotorbauteil verändert wird.

Das Ausgleichsbauteil kann an dem Abgriffbauteil angeordnet sein. Bei dieser Ausführungsform ist das Ausgleichsbauteil an dem Abgriffbauteil montiert. Beispielsweise kann ein Vorsprung oder ein Zapfen des Abgriffbauteils eine Öffnung an dem Ausgleichsbauteil durchgreifen, wenn dieses an dem Abgriffbauteil montiert ist. Hierbei kann vorgesehen sein, dass das Ausgleichsbauteil in mehreren Drehstellungen um das Abgriffbauteil herum jeweils fixierbar ist, zum Beispiel mittels einer lösbaren Feststellschraube, um die Ausgleichswirkung veränderbar bereitzustellen.

An dem Abgriffbauteil kann ein Lagerungsbauteil angeordnet sein, welches eingerichtet ist, zum Abgreifen der drehenden Antriebsbewegung an ein Pleuelbauteil zu koppeln. Die Lagerung des Pleuelbauteils an dem Abgriffbauteil kann beispielsweise unter Verwendung eines Kugel- oder eines Gleitlagers ausgeführt sein.

Das Abgriffbauteil kann an einem Rotorgehäusebauteil angeordnet sein. Zum Beispiel kann das Rotorgehäusebauteil eine rotierende Außenglocke des elektrischen Motors sein. Das Rotorgehäusebauteil kann eine Topf-, Ring- oder eine Scheibenform aufweisen.

Mit dem Gehäuse der Antriebseinrichtung kann ein Handstück gebildet sein, welches von einem Nutzer gegriffen werden kann.

Bei dem Handgerät zum lokalen Aufstechen einer Haut kann die Hautstecheinrichtung mit einem als Einwegmodul ausgeführten Stechmodul gebildet sein. Als Stechmodul ausgeführte Einwegmodule sind als solche in verschiedenen Ausführungsformen bekannt. Sie ermöglichen es, die Hautstecheinrichtung nach einer Benutzung auszutauschen, um so ein Handgerät mit einem hohen Hygienestandard bereitzustellen.

Die Kopplungseinrichtung kann bei dem Handgerät zum lokalen Aufstechen ein Pleuelbauteil aufweisen, welches mit einem den Abgriff bereitstellenden Abgriffbauteil des elektrischen Motors drehentkoppelnd verbunden ist. Das Pleuelbauteil kann mit einer Pleuelstange ausgeführt sein, die antriebseitig ein Pleuelauge aufweist, das in einer gekoppelten Stellung das Abgriffbauteil teilweise oder im Wesentlichen vollständig umgreift. Eine Drehentkopplung kann mittels eines Kugel- oder eines Gleitlagers ausgeführt sein. Ist an dem Abgriffbauteil ein Ausgleichsbauteil zum Bereitstellen einer dem Abgriffbauteil zugeordneten Ausgleichsmasse angeordnet, kann das Ausgleichsbauteil in Bezug auf das Rotorbauteil in einer proximalen Stellung angeordnet sein, wohingegen eine Lagerung des Pleuelbauteils an dem Abgriffbauteil in einer distalen Stellung ausgebildet ist.

Die vorangehend im Zusammenhang mit der Antriebseinrichtung erläuterten weiteren Ausgestaltungen können in Verbindung mit dem Handgerät zum lokalen Aufstechen der Haut entsprechend vorgesehen sein.

### Beschreibung von Ausführungsbeispielen

Im Folgenden werden weitere Ausführungsbeispiele unter Bezugnahme auf Figuren einer Zeichnung erläutert. Hierbei zeigen:
- Fig. 1: eine schematische perspektivische Darstellung eines Handgeräts zum lokalen Aufstechen einer Haut mit einer Antriebseinrichtung und einer hiermit verbundenen Hautstecheinrichtung, die mit einem Antriebsmodul und einem Nadelmodul gebildet sind;
- Fig. 2: eine schematische perspektivische Darstellung des Handgeräts aus Fig. 1, wobei die mit dem Nadelmodul gebildete Hautstecheinrichtung von der Antriebseinrichtung getrennt ist;
- Fig. 3: eine perspektivische Darstellung eines als Außenläufer ausgebildeten elektrischen Motors von schräg vorn;
- Fig. 4: eine schematische perspektivische Darstellung des elektrischen Motors aus Fig. 3 von schräg hinten;
- Fig. 5: eine schematische perspektivische Darstellung des elektrischen Motors aus Fig. 4 von schräg hinten und teilweise im Schnitt, wobei ein Pleuelbauteil an einen exzentrischen Abgriff koppelt;
- Fig. 6: eine schematische perspektivische Darstellung des elektrischen Motors aus Fig. 5 von schräg hinten in einer anderen Drehstellung;
- Fig. 7: eine schematische perspektivische Darstellung eines Endabschnitts des Pleuelbauteils im Detail;
- Fig. 8: eine schematische perspektivische Darstellung des Endabschnitts des Pleuelbauteils aus Fig. 7 im Detail und im Schnitt;
- Fig. 9: eine schematische perspektivische Darstellung eines Ausgleichsbauteils in einstückiger und mehrstückiger Ausführung.

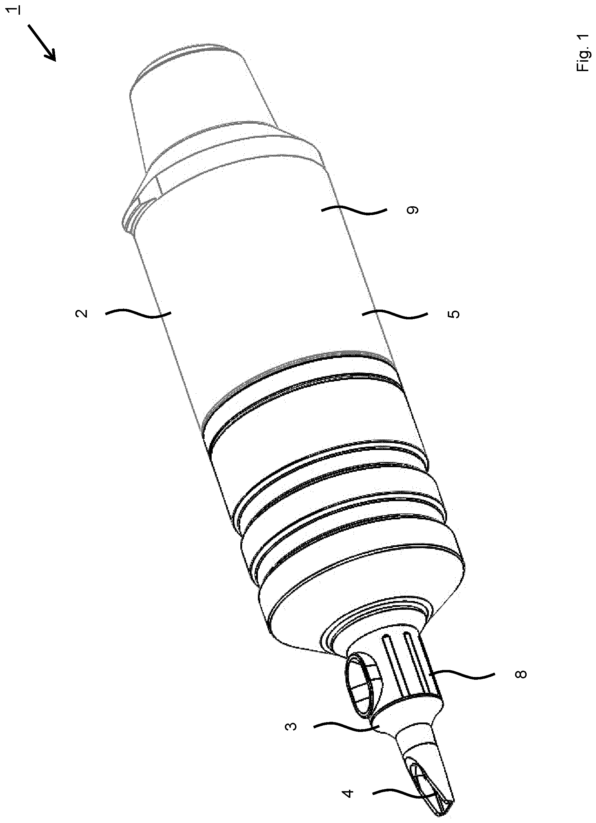

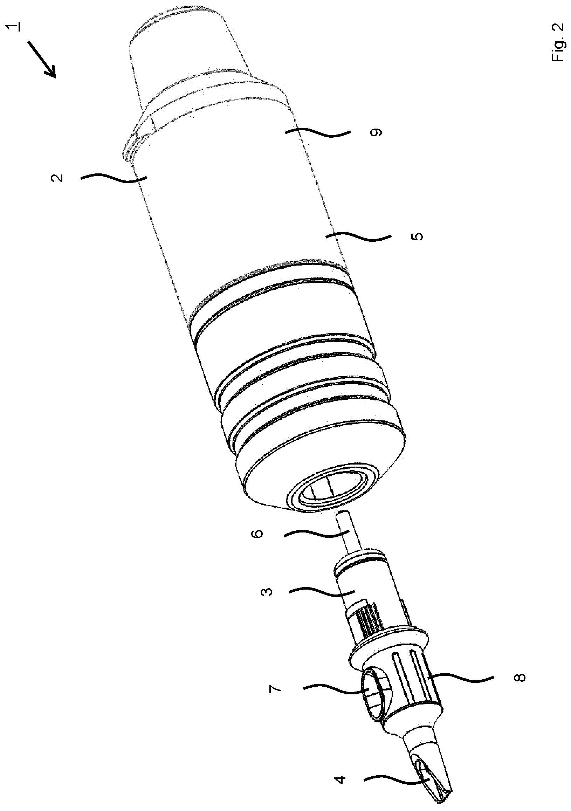

Fig. 1 und 2 zeigen schematische perspektivische Darstellungen eines Handgeräts 1 mit einem eine Antriebseinrichtung bereitstellenden Antriebsmodul 2 und einem hieran gekoppelten und eine Hautstecheinrichtung bereitstellenden Nadelmodul 3, welches gemäß Fig. 2 von dem Handmodul 2 gelöst werden kann, insbesondere nach einer Nutzung des Handgeräts 1 zum lokalen Aufstechen einer Haut, um das als Einwegmodul ausführbare Nadelmodul 3 zu entsorgen.

Das Handgerät 1 ist eingerichtet, eine menschliche oder eine tierische Haut im Betrieb lokal mehrfach mit einer Wiederholfrequenz aufzustechen. Hierzu wird eine Stecheinrichtung mit einer oder mehreren Stechnadeln (nicht dargestellt) durch eine vorderseitige Gehäuseöffnung 4 an dem Nadelmodul 3 vor und zurück bewegt, wie dieses als solches bekannt ist. Um die lineare Vor- und Zurückbewegung der Stecheinrichtung bereitzustellen, ist eine Kopplungseinrichtung (nicht dargestellt) vorgesehen, die beim gezeigten Beispiel in einem Gehäuse 5 des Antriebsmoduls 2 angeordnet und eingerichtet ist, eine von einem elektrischen Motor der Antriebseinrichtung bereitgestellte drehende Antriebsbewegung abzugreifen und in eine Linearbewegung zum Vor- und Zurückbewegen der Stecheinrichtung zu wandeln und die auf die eine oder mehreren Stechnadeln direkt oder indirekt zu koppeln. Somit ist mit der Kopplungseinrichtung ein Wandlungsmechanismus bereitgestellt, wie dies für derartige Handgeräte an sich bekannt ist. Die Stecheinrichtung wird dann mit einer Wiederholfrequenz vor und zurück bewegt.

Über ein mit der einen oder den mehreren Stechnadeln der Stecheinrichtung verbundenes Kopplungsbauteil 6 (vgl. Fig. 2) der mit dem Nadelmodul 3 gebildeten Hautstecheinrichtung wird die lineare Antriebsbewegung auf die Stecheinrichtung übertragen, so dass die eine oder die mehreren Stechnadeln vor und zurückbewegt werden können. Das Kopplungsbauteil 6 kann Teil der Kopplungseinrichtung sein oder getrennt hiervon gebildet sein, um die Linearbewegung abzugreifen und auf die Stecheinrichtung zu geben.

Das Handgerät 1 kann als Tätowiergerät oder als Gerät zum Ausbilden von permanentem Makeup ausgebildet sein. Hierbei kann über eine Öffnung 7 an einem Modulgehäuse 8 ein Farbstoff zugeführt werden, der in die Haut einzubringen ist. Das Handgerät 1 kann aber auch zum lokalen Aufstechen einer Haut ohne Substanzeintrag verwendet werden.

Mit dem Gehäuse 5 der mittels Antriebsmodul 2 bereitgestellten Antriebseinrichtung ist ein Handstück 9 gebildet, welches vom Nutzer gegriffen werden kann, um das Handgerät 1 im Betrieb zu führen.

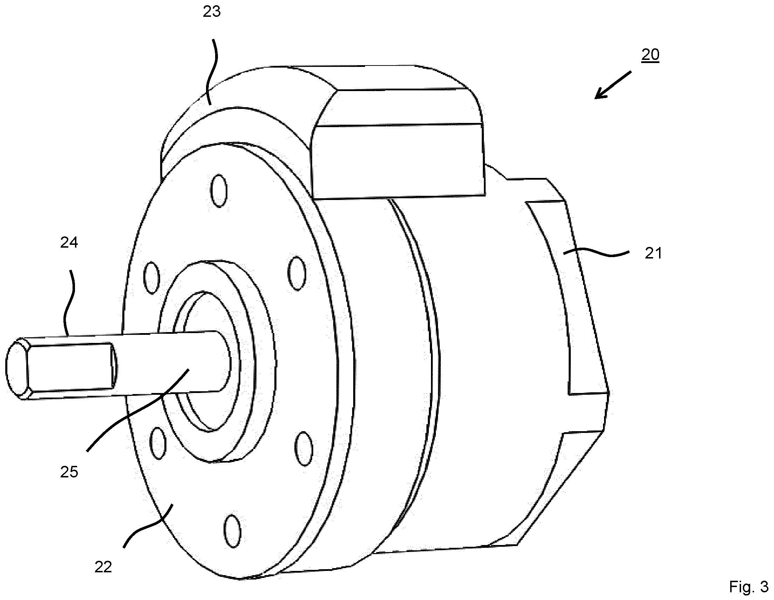

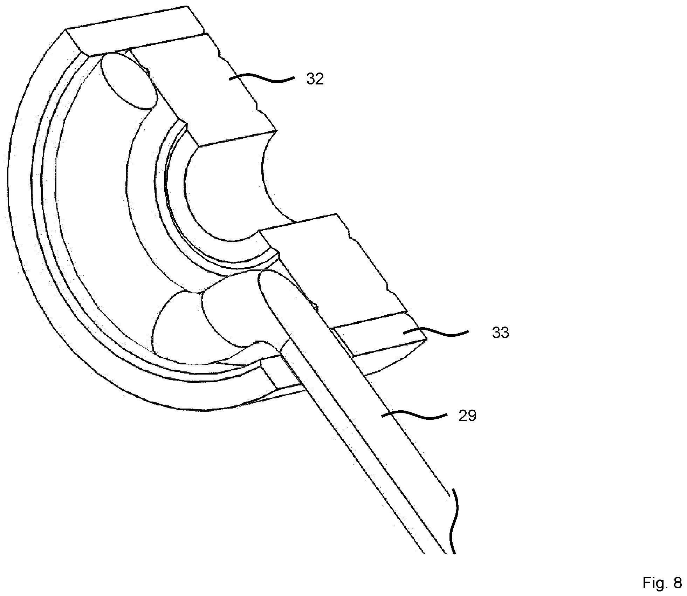

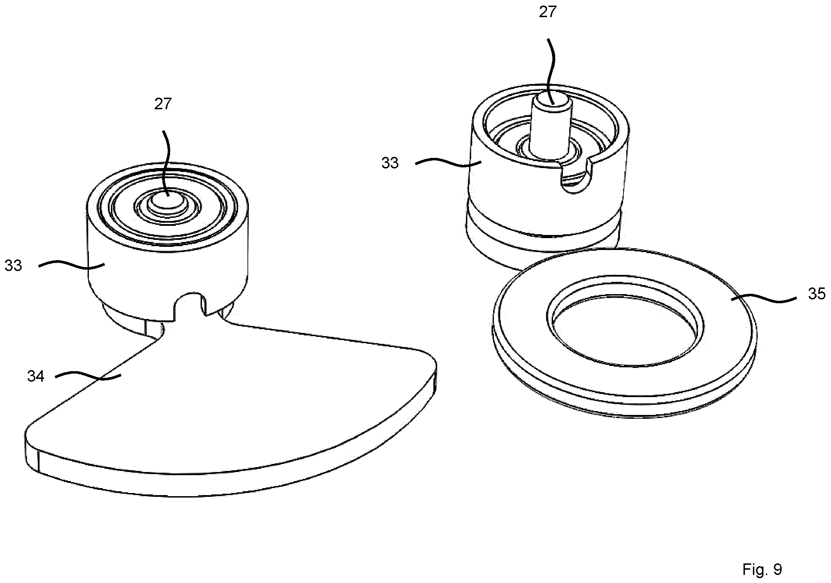

Unter Bezugnahme auf die Fig. 3 bis 9 werden nachfolgend Ausführungsformen für einen als Außenläufer ausgebildeten elektrischen Motor 20 erläutert, der im Gehäuse 5 der mittels Antriebsmodul 2 bereitgestellten Antriebseinrichtung aufgenommen ist, um die drehende Antriebsbewegung bereitzustellen. Beim Außenläufer dreht sich ein außenliegendes Rotorbauteil 21 im Betrieb. Bei der gezeigten Ausführungsform bildet das Rotorbauteil 21 eine rotierende Außenglocke oder ein rotierendes Außengehäuse des elektrischen Motors 20, welches eine Topfform aufweisen kann. Der elektrische Motor 20 kann mit Hilfe eines Andockflansches 22 montiert werden. Elektrische Anschlüsse / Abgriffe sind in einem Block 23 angeordnet.

Das Rotorbauteil 21 dreht sich um eine Motor- oder Rotorachse, welches in Axialrichtung einer Motorwelle 24 ausgebildet ist. Ein abstehender Teil 25 der Motorwelle 24 kann entfallen.

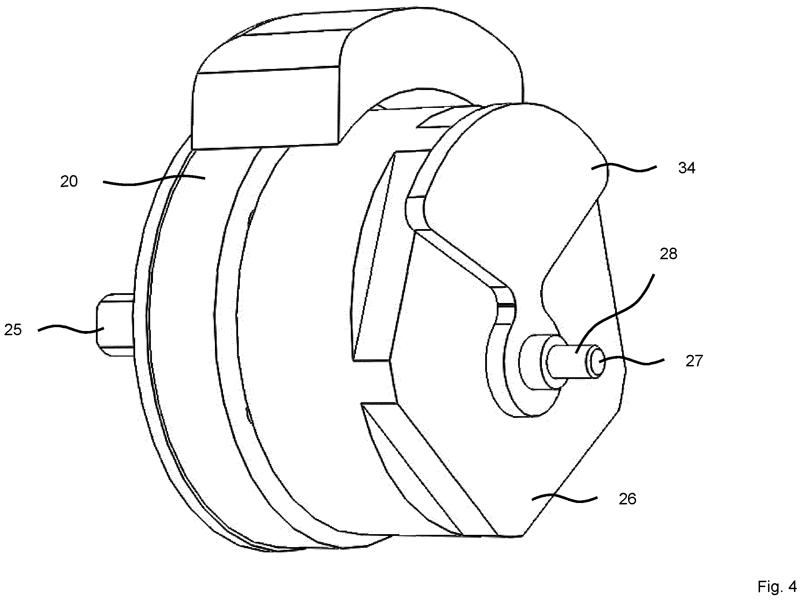

Für den Betrieb des Handgeräts 1 ist vorgesehen, eine exzentrische Drehbewegung mit Hilfe des elektrischen Motors 20 bereitzustellen, die eine Antriebsbewegung für das Vor- und Zurückbewegen der Stechvorrichtung bildet. Hierzu ist gemäß Fig. 4 ein auf einer (rückseitigen) Stirnfläche 26 des Rotorbauteils 21 in Bezug auf die Motorwelle 24 exzentrisch angeordnetes Abgriffbauteil 27 vorgesehen, welches im Ausführungsbeispiel mit einem exzentrischen Kurbelzapfen 28 gebildet ist. Das Abgriffbauteil 27 kann an dem Rotorbauteil 21 beispielsweise angeschweißt oder angeklebt sein. Eine lösbare Montage an dem Rotorbauteil 21 kann vorgesehen sein, insbesondere derart, dass das Abgriffbauteil 27 in mehreren Stellung in unterschiedlichem Abstand zur Motorwelle 24 fixiert werden kann, um so die Exzentrizität einzustellen.

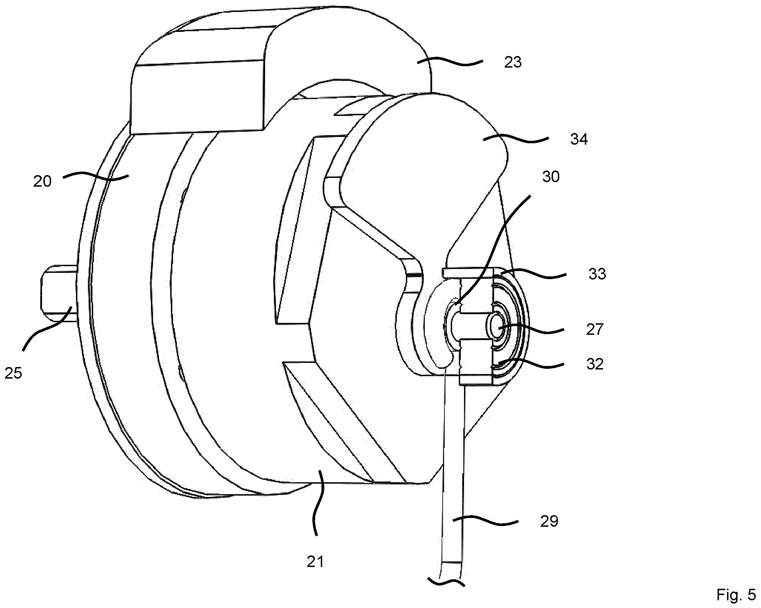

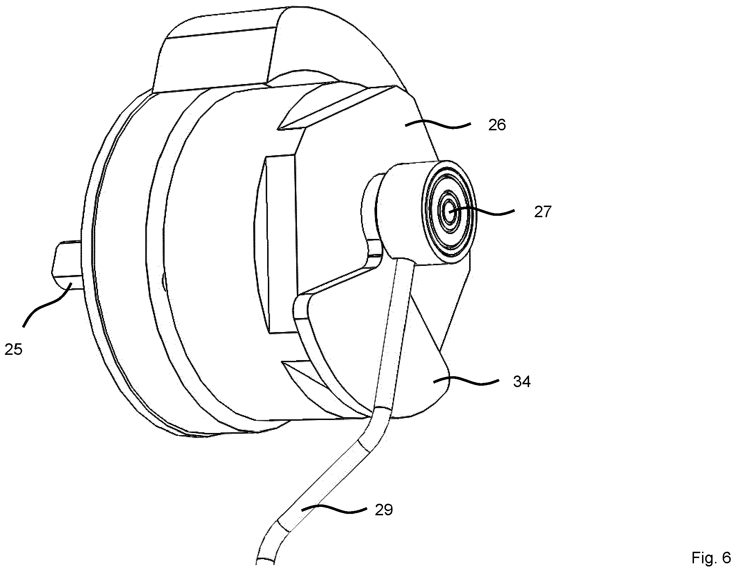

Zum exzentrischen Abgreifen der drehenden Antriebsbewegung koppelt das Abgriffbauteil 27 an ein Pleuelbauteil 29 (vgl. Fig. 5 und 6). Das Pleuelbauteil 29 ist endseitig mit einem Pleuelauge 30 gebildet, welches einen Wellenabsatz 30 auf dem Kurbelzapfen 28 im gezeigten Beispiel im Wesentlichen vollständig umgreift. Das Pleuelbauteil 29 ist mit dem Abgriffbauteil 27 drehentkoppelt verbunden. Hierzu ist ein Kugellager 32 in einem umgebenden Außenring 33 vorgesehen, welches am exzentrisch angeordneten Kurbelzapfen 28 oberhalb des Pleuelbauteils 29 angeordnet ist.

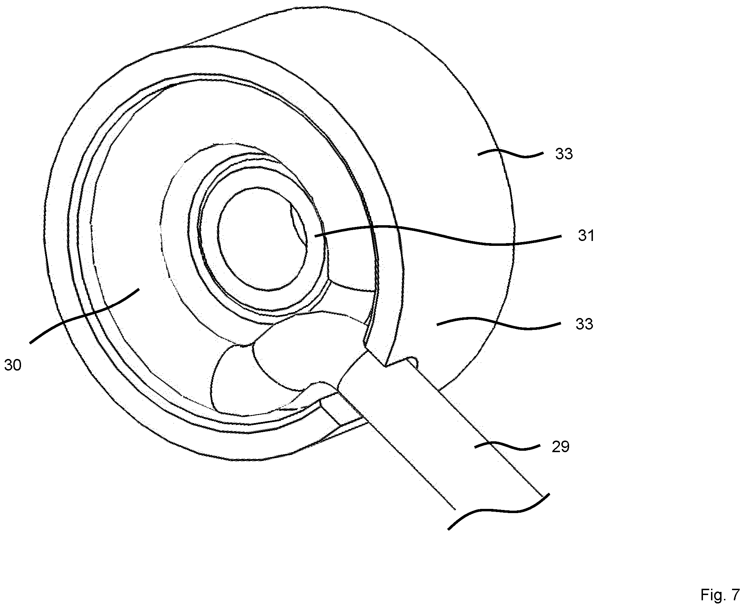

Insbesondere gemäß den Fig. 4 bis 6 ist an dem Abgriffbauteil 27 weiterhin ein Ausgleichsbauteil 34 vorgesehen, welches eine dem Abgriffbauteil 27 zugeordnete Ausgleichsmasse für eine Auswuchtung bereitstellt. Für das Ausgleichsbauteil 34 zeigen die Fig. 7 bis 9 die einstückige sowie eine alternative mehrstückige Ausbildung. Bei der mehrstückigen Ausführung kann ein Normbauteil 35 auf dem Rotorbauteil 21 außenseitig angeklebt oder angeschweißt sein.

Es kann vorgesehen sein, dass das Ausgleichsbauteil auf dem Rotorbauteil 21 verlagerbar angeordnet ist, derart, dass ein Abstand zwischen einer Rotorachse des elektrischen Motors 20 und dem Abgriffbauteil in radialer Richtung veränderbar ist, so dass die Exzentrizität des Abgriffbauteils einstellbar ist. Im Zusammenwirken mit dem Pleuelbauteil kann so ein Hub für die Vor- und Zurückbewegung der Stecheinrichtung eingestellt werden.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Antriebseinrichtung für ein Handgerät (1) zum lokalen Aufstechen einer Haut, mit:
- einem Gehäuse (5), welches mit einem Modulgehäuse (8) einer Hautstecheinrichtung verbindbar ist; und
- einer elektrischen Antriebsvorrichtung, die in dem Gehäuse (5) angeordnet ist und ein Abgriffbauteil (27) aufweist, welches eingerichtet ist, einen Abgriff zum Abgreifen einer mittels der elektrischen Antriebsvorrichtung erzeugten drehenden Antriebsbewegung für die Hautstecheinrichtung bereitzustellen;
**dadurch gekennzeichnet, dass** die elektrische Antriebsvorrichtung einen als Außenläufer mit einem rotierenden Rotorbauteil (21) ausgebildeten elektrischen Motor (20) aufweist, bei dem das Abgriffbauteil (27) an dem Rotorbauteil (21) und in Bezug auf eine Rotorachse exzentrisch angeordnet ist.

2. Antriebseinrichtung nach Anspruch 1, **dadurch gekennzeichnet, dass** das Abgriffbauteil (27) auf einer Stirnfläche (26) des Rotorbauteils (21) angeordnet ist.

3. Antriebseinrichtung nach Anspruch 1 oder 2, **dadurch gekennzeichnet, dass** das Abgriffbauteil (27) an dem Rotorbauteil (21) verlagerbar angeordnet ist, derart, dass eine Exzentrizität des Abgriffs in Bezug auf die Rotorachse einstellbar ist.

4. Antriebseinrichtung nach mindestens einem der vorangehenden Ansprüche, **dadurch gekennzeichnet, dass** das Abgriffbauteil (27) an dem Rotorbauteil (21) einstückig angeformt ist.

5. Antriebseinrichtung nach mindestens einem der Ansprüche 1 bis 3, dadurch **ge- kennzeichnet**, dass das Abgriffbauteil (27) an dem Rotorbauteil (21) lösbar angeordnet ist.

6. Antriebseinrichtung nach mindestens einem der vorangehenden Ansprüche, **gekennzeichnet durch** eine dem Abgriffbauteil (27) zugeordnete Ausgleichsmasse.

7. Antriebseinrichtung nach Anspruch 6, **dadurch gekennzeichnet, dass** die Ausgleichsmasse mit einem Ausgleichsbauteil (34) gebildet ist, welches an dem Rotorbauteil (21) montiert ist.

8. Antriebseinrichtung nach Anspruch 7, **dadurch gekennzeichnet, dass** das Ausgleichsbauteil (34) an dem Abgriffbauteil (27) angeordnet ist.

9. Antriebseinrichtung nach mindestens einem der vorangehenden Ansprüche, **dadurch gekennzeichnet, dass** an dem Abgriffbauteil (27) ein Lagerungsbauteil angeordnet ist, welches eingerichtet ist, zum Abgreifen der drehenden Antriebsbewegung an ein Pleuelbauteil (29) zu koppeln.

10. Antriebseinrichtung nach mindestens einem der vorangehenden Ansprüche, **dadurch gekennzeichnet, dass** das Abgriffbauteil (27) an einem Rotorgehäusebauteil angeordnet ist.

11. Antriebseinrichtung nach mindestens einem der vorangehenden Ansprüche, **dadurch gekennzeichnet, dass** mit dem Gehäuse (5) ein Handstück (9) gebildet ist.

12. Handgerät (1) zum lokalen Aufstechen einer Haut, mit:
- einer Antriebseinrichtung nach mindestens einem der vorangehenden Ansprüche;
- einer Hautstecheinrichtung mit einem Modulgehäuse (8), welche mit der Antriebseinrichtung lösbar verbunden ist und bei der im Betrieb eine Stecheinrichtung in einer vorderseitigen Modulgehäuseöffnung (5) vor und zurück bewegt werden kann; und
- einer Kopplungseinrichtung, die mit der Antriebseinrichtung und / oder der Hautstecheinrichtung gebildet und eingerichtet ist, eine in der Antriebseinrichtung bereitgestellte drehende Antriebsbewegung am Abgriff eines als Außenläufer ausgebildeten elektrischen Motors (20) abzugreifen und in eine Linearbewegung zu wandeln und diese auf die Stecheinrichtung zu koppeln.

13. Handgerät nach Anspruch 12, **dadurch gekennzeichnet, dass** die Hautstecheinrichtung mit einem als Einwegmodul ausgeführten Nadelmodul (3) gebildet ist.

14. Handgerät nach Anspruch 12 oder 13, **dadurch gekennzeichnet, dass** die Kopplungseinrichtung ein Pleuelbauteil (29) aufweist, welches mit einem den Abgriff bereitstellenden Abgriffbauteil (27) des elektrischen Motors (20) drehentkoppelnd verbunden ist.

15. Handgerät nach mindestens einem der Ansprüche 12 bis 14, dadurch **gekenn- zeichnet**, dass mit einem Gehäuse (5) der Antriebseinrichtung ein vom Nutzer greifbares Handstück (9) gebildet ist.

## Claims

1. A drive device for a hand-held device (1) for locally piercing skin, with:
- a housing (5) which can be connected to a module housing (8) of a skin piercing device; and
- an electric drive means which is disposed in the housing (5) and has a tapping component (27) which is configured to provide a tap for the skin piercing device for tapping a rotary drive movement generated by the electric drive means;
**characterized in that** the electric drive means comprises an electric motor (20) provided as an external rotor motor with a rotating rotor component (21), in which the tapping component (27) is disposed on the rotor component (21) and is eccentric with respect to a rotor axis.

2. The drive device as claimed in claim 1, **characterized in that** the tapping component (27) is disposed on an end face (26) of the rotor component (21).

3. The drive device as claimed in claim 1 or claim 2, **characterized in that** the tapping component (27) is displaceably disposed on the rotor component (21) such that an eccentricity of the tap with respect to the rotor axis can be adjusted.

4. The drive device as claimed in at least one of the preceding claims, **characterized in that** the tapping component (27) is integrally formed on the rotor component (21).

5. The drive device as claimed in at least one of claims 1 to 3, **characterized in that** the tapping component (27) is detachably disposed on the rotor component (21).

6. The drive device as claimed in at least one of the preceding claims, **characterized by** a counterbalance mass associated with the tapping component (27).

7. The drive device as claimed in claim 6, **characterized in that** the counterbalance mass is formed with a counterbalance component (34) which is mounted on the rotor component (21).

8. The drive device as claimed in claim 7, **characterized in that** the counterbalance component (34) is disposed on the tapping component (27).

9. The drive device as claimed in at least one of the preceding claims, **characterized in that** a bearing component is disposed on the tapping component (27), the bearing component being configured to couple to a connecting rod component (29) in order to tap the rotary drive movement.

10. The drive device as claimed in at least one of the preceding claims, **characterized in that** the tapping component (27) is disposed on a rotor housing component.

11. The drive device as claimed in at least one of the preceding claims, **characterized in that** a handpiece (9) is formed with the housing (5).

12. A hand-held device (1) for locally piercing skin, with:
- a drive device according to at least one of the preceding claims;
- a skin piercing device with a module housing (8) which skin piercing device is detachably connected to the drive device and in which, during operation, a piercing device can be moved forwards and backwards in a module housing opening (5) at the front; and
- a coupling device which is formed with the drive device and/or the skin piercing device and is configured to tap a rotary drive movement provided in the drive device at the tap of an electric motor (20) provided as an external rotor motor and convert it into a linear movement and couple it to the piercing device.

13. The hand-held device as claimed in claim 12, **characterized in that** the skin piercing device is formed with a needle module (3) configured as a disposable module.

14. The hand-held device as claimed in claim 12 or claim 13, **characterized in that** the coupling device has a connecting rod component (29) which is connected in a rotationally decoupling manner to a tapping component (27) of the electric motor (20) providing the tap.

15. The hand-held device as claimed in at least one of claims 12 to 14, **characterized in that** a handpiece (9) which can be gripped by the user is formed with a housing (5) of the drive device.

## Revendications

1. Dispositif d'entraînement pour appareil portatif (1) de piqûre locale de la peau, comportant :
- un boîtier (5) qui peut être relié à un boîtier de module (8) d'un dispositif de piqûre cutanée ; et
- un dispositif d'entraînement électrique qui est disposé dans le boîtier (5) et qui comporte un élément de captation (27) qui est conçu pour permettre une captation destinée à capter un mouvement d'entraînement rotatif généré par le dispositif d'entraînement électrique pour le dispositif de piqûre cutanée ;
**caractérisé en ce que** le dispositif d'entraînement électrique présente un moteur électrique (20) réalisé sous la forme d'un rotor extérieur avec un élément de rotor rotatif (21), dans lequel l'élément de captation (27) est disposé de manière excentrique sur l'élément de rotor (21) et par rapport à un axe de rotor.

2. Dispositif d'entraînement selon la revendication 1, **caractérisé en ce que** l'élément de captation (27) est disposé sur une surface frontale (26) de l'élément de rotor (21).

3. Dispositif d'entraînement selon la revendication 1 ou 2, **caractérisé en ce que** l'élément de captation (27) est disposé sur l'élément de rotor (21) de manière à pouvoir coulisser, de telle sorte qu'une excentricité de la captation peut être réglée par rapport à l'axe du rotor.

4. Dispositif d'entraînement selon au moins l'une quelconque des revendications précédentes, **caractérisé en ce que** l'élément de captation (27) est réalisé d'une seule pièce sur l'élément de rotor (21).

5. Dispositif d'entraînement selon au moins l'une des revendications 1 à 3, **caractérisé en ce que** l'élément de captation (27) est disposé de manière amovible sur l'élément de rotor (21).

6. Dispositif d'entraînement selon au moins l'une des revendications précédentes, **caractérisé par** une masse de compensation associée à l'élément de captation (27).

7. Dispositif d'entraînement selon la revendication 6, **caractérisé en ce que** la masse de compensation est formée avec un composant de compensation (34) qui est monté sur l'élément de rotor (21).

8. Dispositif d'entraînement selon la revendication 7, **caractérisé en ce que** le composant de compensation (34) est disposé sur l'élément de captation (27).

9. Dispositif d'entraînement selon au moins l'une des revendications précédentes, **caractérisé en ce qu'**est disposé sur l'élément de captation (27) un élément palier qui est conçu pour être couplé à un élément bielle (29) pour capter le mouvement d'entraînement rotatif.

10. Dispositif d'entraînement selon au moins l'une des revendications précédentes, **caractérisé en ce que** l'élément de captation (27) est disposé sur un élément boîtier de rotor.

11. Dispositif d'entraînement selon au moins l'une des revendications précédentes, **caractérisé en ce qu'**une poignée (9) est formée avec le boîtier (5).

12. Appareil portatif (1) pour piquer localement la peau, comportant :
- un dispositif d'entraînement selon au moins l'une des revendications précédentes ;
- un dispositif de piqûre cutanée comportant un boîtier de module (8), qui est connecté de manière dissociable au dispositif d'entraînement et pour lequel, en cours de fonctionnement, un dispositif de piqûre peut être déplacé d'avant en arrière dans une ouverture frontale du boîtier de module (5) ; et
- un dispositif d'accouplement, qui est formé avec le dispositif d'entraînement et/ou le dispositif de piqûre cutanée et est conçu pour capter un mouvement d'entraînement rotatif exécuté dans le dispositif d'entraînement à la prise d'un moteur électrique (20) conçu comme un rotor externe et le convertir en un mouvement linéaire et pour coupler celui-ci au dispositif de piqûre.

13. Appareil à main selon la revendication 12, **caractérisé en ce que** le dispositif de piqûre cutanée est formé avec un module d'aiguille (3) réalisé sous la forme d'un module jetable.

14. Appareil à main selon la revendication 12 ou 13, **caractérisé en ce que** le dispositif d'accouplement présente un élément bielle (29) qui est relié à un élément de captation (27) permettant la captation avec désaccouplement de rotation du moteur électrique (20).

15. Appareil à main selon au moins l'une des revendications 12 à 14, **caractérisé en ce qu'**une poignée (9) pouvant être saisie par l'utilisateur est formée avec un boîtier (5) du dispositif d'entraînement.
